# EUROPÄISCHE PATENTSCHRIFT

(11) **EP 1 503 772 B1**
(45) Veröffentlichungstag und Bekanntmachung des Hinweises auf die Patenterteilung: **17.12.2008**
(21) Anmeldenummer: 03720506.9
(22) Anmeldetag: 19.04.2003
(51) Int. Cl.: A61K 36/00, A61K 8/97, A61K 9/08, A23L 1/076

(54) **SPOROPOLLENIN ENTHALTENDE LÖSLICHE ZUSAMMENSETZUNG UND VERWENDUNG**
SOLUBLE COMPOSITION CONTAINING SPOROPOLLENIN AND THE USE THEREOF
COMPOSITION SOLUBLE CONTENANT DE LA SPOROPOLLENINE ET UTILISATION DE LADITE COMPOSITION

(30) Priorität: 13.05.2002 DE 10221212
(43) Veröffentlichungstag der Anmeldung: 09.02.2005
(73) Patentinhaber: Maack, Andreas, 31717 Nordsehl (DE); Maack, Ines, 31717 Nordsehl (DE)
(72) Erfinder: MAACK, Andreas, D-31717 Nordsehl (DE)
(74) Vertreter: Sieckmann, Ralf
(86) Internationale Anmeldenummer: PCT/EP2003/004111
(87) Internationale Veröffentlichungsnummer: WO 2003/094942

(56) Entgegenhaltungen:
- EP-A- 0 019 925
- DE-A- 19 902 724
- US-A- 4 737 360
- E. DOMINGUEZ ET AL.: "Pollen sporopollenin: degradation and structural elucidation" SEXUAL PLANT REPRODUCTION, Bd. 12, Nr. 3, 21. September 1999 (1999-09-21), Seiten 171-178, XP002252188 Heidelberg (DE)
- DATABASE WPI Thomson Scientific, London, GB; AN 1990-288169 & KR 890 003 692 B (LEE P H) 30. September 1989 (1989-09-30)
- JUNGFERMANN ET AL.: "Solution of sporopollenin and reaggregation of a sporopollenin-like material: A new approach in the sporopollenin research" J. PLANT PHYSIOL., Bd. 151, 1997, Seiten 513-519,
- ERSOZ ET AL.: "Ion exchange equilibria of heavy metals in aqueous solution on new chelating resins of sporopollenin" REACTIVE POLYMERS, 1995, Seiten 195-202,

## Beschreibung

Die vorliegende Erfindung betrifft eine Sporopollenin enthaltende Zusammensetzung und Verwendung, wie im Nachfolgenden erörtert.

Unter einem Sporopollenin versteht man beispielsweise gemäß Römpp - Lexikon Naturstoffe, 1997, Seite 604, Stichwort Sporopollenin: "in Blütenstaub (Pollen) vorkommende Polyterpene, die möglicherweise durch oxydative Polymerisierung aus Carotinoiden und deren Estern entstehen".

Diese Verbindung hat als Polyterpen allerdings keine Gemeinsamkeiten mit entsprechenden Carotinmonomeren, wie z.B. dem Beta-Carotin.

Sporopollenin findet sich weiterhin in sehr kleinen Mengen in Sporen und Pollen von Pflanzen, beispielsweise Farnen, Bärlapp, Blütenpollen, aber sind auch Bestandteil von speziellen Algenarten. Die Herstellung von Sporopollenin wird in der deutschen Offenlegungsschrift 199 02 724 in Form von Mikrokapseln und ihre Anwendung als mechanische Stützphase für die Verkapselung von Stoffen und Zellen beschrieben, wobei das Sporopollenin allerdings in den Kapseln als Dispersion und nicht als Lösung vorliegt.

R. Wiermann, 1H-NMR-Analysis of Sporopollenine from Typha Angustifolia in Phytochemistry Band 50 (1999), 1095-1098, berichtet bei der Analytik über die Unlöslichkeit und von Sporopollenin, welches in nur sehr geringen Mengen herstellbar ist. Die Autoren berichten, dass sie das Sporopollenin nur in Mengen von 5 mg pro 5 ml in hochpolaren Lösemitteln wie Aminoethanol gelöst bekommen.

Der vorliegenden Erfindung liegt die Aufgabe zugrunde, eine Sporopollenin enthaltende Zusammensetzung bereitzustellen, die entweder allein oder in Kombination mit biologisch aktiven Wirkstoffen in Lösung gebracht werden kann.

Diese Aufgabe wird dadurch gelöst, dass Sporopollenin mit einer speziellen Menge wenigstens eine wie in dem Ansprüchen definierten Emulgators und einer speziellen Menge wenigstens eines hydrophilen Lösemittels kontaktiert und damit in an sich bekannter Weise durch Erwärmen oberhalb von Raumtemperatur, d.h. von 10 - 25° C, in Lösung gebracht wird.

Die vorliegende Erfindung betrifft damit zunächst eine Sporopollenin enthaltende Zusammensetzung, die dadurch gekennzeichnet ist, dass sie als Lösung vorliegt und neben 0,01 bis 12 Gew.%, vorzugsweise 0,1 bis 5 Gew.% Spooopollenin, 0,01 bis 17. Gew.-%, vorzugsweise 0,1 bis 10 Gew.% wenigstens eines wie in den Ansprüchen definierten Emulgators und 71,00 bis 99,98 Gew.%, vorzugsweise 85 bis 99,80 Gew.-% wenigstens eines hydrophilen Lösemittels aufweist.

Unter einer Lösung im Sinne der vorliegenden Erfindung versteht man eine meist je nach Konzentration um eine mehr oder weniger hell bis leicht gelb gefärbte transparente Flüssigkeit mit mehr oder weniger hoher Viskosität, die sich aber im wesentlichen aus der Viskosität des Lösemittels selbst ergibt.

Nach einer bevorzugten Ausführungsform der vorliegenden Erfindung weist diese Zusammensetzung als Emulgator eine hydrolisierbare Etherverbindung auf.

Sofern es sich bei der hydrolisierbaren Verbindung um eine Esterverbindung handeln sollte, kann diese entweder ein Emulgator mit einer Alkohol-Komponente oder ein Emulgator mit mehreren unterschiedlichen Alkohol-Komponenten sein.

Sofern es sich bei der bevorzugten Ausführungsform des Emulgator um einen Emulgator mit einer Alkohol-Komponente handelt, so kann dieser Alkohol ein- oder mehrwertig sein und gegebenenfalls weitere funktionelle Gruppen enthalten.

Beispiele für Emulgatoren mit einer einwertigen Alkohol-Komponente sind solche von Fettalkoholen abgeleitete, einwertige Alkohole, beispielsweise solche, die von 2-Ethylhexanol, von Dodecanol, von Octadecanol abgeleitet sind. Beispielhaft wird hierbei auf den Dioctyl-sulfobernsteinsäure-diester (Na-Salz), das Natriumsalz der Laurylschwefelsäure, das Natriumsalz des Stearyl-fumarsäure, das Stearyltartrat und das Stearylcitrat hingewiesen.

Weitere Beispiele für hydrolisierbare Esterverbindungen sind Emulgatoren mit einer zweiwertigen Alkohol-Komponente oder deren Kondensate, beispielsweise auf Basis von Glykol, Polyglykol, 1,2-Propandiol, Poly-1,2-Propandiol, 1 ,3-Butandiol sowie Glykol-1,2-Propandiol-Mischkondensate. Beispiele hierfür sind Glykol-mono- und -diester, Polyoxyethylen-fettsäureester, wie sie unter dem Handelsnamen "MYRJ" sowie als Propylenglykol-fettsäureester vertrieben werden.

Weitere Beispiele für hydrolisierbare Esterverbindungen von dreiwertigen Alkoholen und deren Kondensate sind solche auf Basis von Glycerin, sei es von Speisefettsäuren als einziger Säurekomponente, d.h. Fettsäure-mono oder -diclyceride, modifizierten Fettsäuren allein oder mit Speisefettsäuren, Sojafettsäuren, die thermisch oxidiert sind, oder sonstigen Säuren als Säurekomponente neben Fettsäuren wie Phosphorsäure, Phosphatidsäuren, Lecithine, Essigsäure, Milchsäure, Bernsteinsäure, Weinsäure, 0,0-Diacetylweinsäure, Citronensäure, Sulfoessigsäure. Derartige Produkte werden unter den Bezeichnungen Emulgator-yn, Emphos (Na-Salz), Sojalecithin, Eilecithin, fraktioniertes Lecithin, Aceto(mono)glyceride, acetylierte Mono-/Diglyceride, Monoglycerid-acetat, Lactoglyceride, lactylierte Mono-/Diglyceride, Monoglycerid-lactat, Succino-glyceride, succinylierte Monoglyceride, Weinsäure-glyceride, Monoglyceridtartrat, Monoglycerid-diacetyltartrat, Citroglyceride, Citronensäüre(mon)glycerid-ester sowie ethoxylierte Mono- und Diglyceride, beispielsweise unter der Bezeichnung "Polyglycerat" vertrieben.

Weitere Beispiele für dreiwertige Alkohole und deren Kondensate sind Derivate von Polyglycerin, sei es Speisefettsäuren als einzige Karbonsäurekomponente in Form von Polyglycerin-fettsäureester, modifizierte Fettsäuren wie Polyglycerin-polyricinoleat, Polyglycerinester dimerisierter Sojaöl-Fettsäuren sowie sonstige Säuren als Säurekomponente neben Fettsäuren, wie beispielsweise Weinsäure-polyglyceride.

Weitere Beispiele für hydrolisierbare Ester mit Alkohol-Komponenten sind solche vierwertigen Alkohole, wie beispielsweise Pentaerythrit, solche fünfwertigen Alkohole, solche sechswertigen Alkohole wie Sorbit, -mono- und -dianhydride wie Sorbitan-fettsäureester , welche unter den Bezeichnungen "Span" vertrieben werden, weiter Polyoxyethylen-sorbitane wie Polyoxyethylen-sorbitan-fettsäureester, welche unter den Bezeichnungen "Polysorbat" oder "Tween" vertrieben werden.

Weitere Beispiele für hydrolisierbare Esterverbindungen sind solche mit einem Kohlehydrat als Alkohol-Komponente, sei es Saccharose oder sei es ein Saccharose-fettsäureester.

Weitere Beispiele für hydrolisierbare Esterverbindungen sind Hydroxycarbonsäuren als Alkohol-Komponente, sei es Milchsäure und deren Kondensate, wie Lactylfettsäureester oder Stearoyl-2-lactylmilchsäure und schließlich Polyol-glykoside als Alkohol-Komponente in Form von 1 ,2-Propandiol-glykosid-fettsäureestern und Glyceringlykosid-fettsäureestern.

Schließlich kann die hydrolisierte Estergruppe auch mehrere unterschiedliche Alkohol-Komponenten aufweisen, wobei die unterschiedliche Alkohol-Komponente im selben Reaktionsgemisch eine einzige Alkohol-Komponente im selben Estermolekül sein kann, wie z.B. Fettsäuren als Carbonsäure-Komponente wie Polyglykol-/Glycerinester von Fettsäuren, 1,2-Propandiol-/Glycerinester von Fettsäuren, Sorbitan-/Glycerinester von Fettsäuren, Polyoxyethylen-sorbitan-/Glycerinester von Fettsäuren, Saccharose-/Glycerinester von Fettsäuren können aber auch neben der Fettsäure noch eine weitere Carbonsäure-Komponente enthalten, wie beispielsweise 1,2-Propandiol-/Glycerinmischester von Fettsäuren und Milchsäure.

Nach einer weiteren bevorzugten Ausführungsform handelt es sich bei dem Emulgator um einen hydrophilen nichtionischen Emulgator, der alternativ auch durch den HLB Wert eines Emulgators definiert werden kann, d.h. über das Maß der hydro-lipophilen Verhältnisse. Hier handelt es sich um die relative Stärke der polaren Gruppen und des apolaren Restes und die dadurch bedingte Affinität des Emulgators zu Wasser. Es wurde erstaunlicherweise gefunden, dass erfindungsgemäß solche nichtionischen Emulgatoren eingesetzt werden können, welche einen HLB Wert von 8 - 18, insbesondere aber 10 -17 aufweisen.

Als weitere Komponente wird ein hydrophiles Lösemittel eingesetzt, welches Wasser oder ein Alkanol mit 1 - 22 Kohlenstoffatomen sein. Dies kann sowohl ein einwertiger Alkohol sein, wobei Ethanol, n-Propanol und Iso-Propanol bevorzugt sind.

Weiter kann es sich hierbei auch um einen mehrwertigen Alkohol handeln, also einen Alkohol, der mindestens zwei alkoholische Hydroxylgruppen im Molekül besitzt. Beispiele hierfür sind Diole, Glykole, Glycerin, aber auch Zuckeralkohole, Polyalkylenglykole wie Polyethylenglykol, Polyether- und Polyesterpolyole.

Weiterer Gegenstand der vorliegenden Erfindung ist die Bereitstellung einer Sporopollenin enthaltenden Zusammensetzung, wobei das Sporopollenin hierbei als Feststoff eingesetzt wird. Insbesondere vorteilhaft hat sich erwiesen, diesen Sporopollenin enthaltenden Feststoff zur kosmetischen Behandlung der Haut, insbesondere der Gesichtshaut, gegen altersbedingte und 1 oder sonnenbedingte Hautfaltenbildung zu verwenden. Alternativ ist es auch möglich, den Sporopollenin enthaltenden Feststoff als Nahrungs- oder Futtermittel Wirbeltieren, insbesondere Säugetieren, aber auch Menschen zu verabreichen.

Weiterer Gegenstand der vorliegenden Erfindung ist die Bereitstellung von biologischen Zusammensetzungen, die die vorgenannten Sporopollenin-Lösungen zusammen mit üblichen Hilfs- und Zusatzstoffen enthalten.

Dies sind zunächst entsprechende kosmetische Zusammensetzungen, die eine derartige Sporopollenin-Lösung in Form eines Öls, einer Salbe, einer Creme, eines Gels eingebettet in Liposomen oder als fester Stift enthalten.

Nach einer weiteren bevorzugten Ausführungsform kann die Zusammensetzung als hydrophile Salbe vorliegen. Für ihren Einsatz als Oberflächensalbe kommt bei hydrophilen Salben als Salbengrundlage Makrogele, d.h. Polyethylenglykol oder Polyoxyethylenpolymerisate in Betracht. In diesem Zusammenhang verweisen wir beispielhaft auf die Monografie "Arzneimittelformlehre" von Frau Schöffling, Stuttgart 1998, Seite 310 - 319 sowie 328 - 337 und die dort beschriebenen Substanzen und Herstellmethoden und auf Bauer, Pharmazeutische Technologie, Stuttgart 1986, Seite 312 - 323 sowie 330 bis 331.

Nach einer weiteren bevorzugten Ausführungsform kann die Zusammensetzung als hydrophile (O/W) Creme vorliegen. Wiederum verweisen wir beispielhaft auf die vorgenannte Monografie von Schöffling, Seite 320 - 324 und auf Bauer, Seite 323 - 328 sowie 331 - 332.

In einer weiteren bevorzugten Ausführungsform kann die Zusammensetzung als Gel vorliegen. Unter hydrophile Gele fallen Hydrogele auf Basis von Polyacrylaten und Acrylsäuren und auf Basis von Celluloseethern. Wir verweisen hierzu beispielhaft auf die vorgenannte Monografie von Schöffling, Seite 324 - 327 sowie auf Bauer, Seite 329 -330.

Nach einer weiteren bevorzugten Ausführungsform kann die Zusammensetzung als Öl auf Basis wenigstens eines Fettsäureesters eines mineralischen oder fetten Öls vorliegen. Derartige Substrate werden üblicherweise als Hautöle bezeichnet. Wir verweisen beispielsweise auf die Übersicht bei G.A. Nowak, "Die kosmetischen Präparate", 2. Auflage, Seite 657ff.

Wie dem Fachmann auf diesem Gebiete bekannt, können alle vorgenannten pharmazeutischen oder kosmetischen galenischen Darreichungsformen in an sich bekannter Weise aus den entsprechenden wässrigen und / oder Fettphasen durch Vermischen und gegebenenfalls Homogenisieren mit den Sporopollenin-LÖsungen erhalten werden.

Nach einer weiteren bevorzugten Ausführungsform fallen unter die erfindungsgemäßen Zusammensetzungen auch pharmazeutische Zusammensetzungen, die die vorgenannte Sporopolleninlösung in Form eines Öl, einer Creme oder gegebenenfalls einer Kapsel enthalten.

Bezüglich der galenischen Formen des Öls und der Cremes verweisen wir auf die vorgenannten Literaturstellen von Schöffling und Bauer. Darüber hinaus können pharmazeutische Zusammensetzungen auch in verkapselter Form eingesetzt werden. Sofern hier die erfindungsgemäßen Sporopolleninlösungen eingesetzt werden sollen, kommen die entsprechenden Gelatineweichkapseln zum Einsatz. Wir verweisen insofern auf die entsprechende Literatur zur Galenik von Schöffling auf Seite 164-179.

Alternativ kann auch ein Einsatz von sporopolleninhaltigen Zusammensetzungen in fester Form geschehen, beispielsweise durch eine Verabreichung in Gelatinesteckkapseln. Wir verweisen insofern auf die entsprechende Galenik hierzu in Schöffling, Seite 168 -178.

Nach einer weiteren bevorzugten Ausführungsform können die sporopolleninhaltigen Lösungen der vorgenannten Art zur kosmetischen Behandlung der Haut, insbesondere der Gesichtshaut gegen altersbedingte und / oder sonnenbedingte Hautfaltenbildung eingesetzt werden.

Nach einer weiteren bevorzugten Ausführungsform können die vorgenannten Sporopollenin enthaltenden Lösungen als Nahrungs(ergänzungs)mittel für Wirbel-tiere, insbesondere aber für Säugetiere, Haus- und Nutztiere und auch für Menschen eingesetzt werden.

Nach einer besonderen Ausführungsform hierzu kann das so eingesetzte Sporopollenin nicht nur als Lösung, sondern auch als Feststoff eingesetzt werden.

Die vorliegende Erfindung wird nachfolgend durch Herstellungs- und Ausführungsbeispiele näher erläutert. Die hierbei gegebenen Verhältnisse sind stets als Gewichtsverhältnisse anzusehen.

### Herstellungsbeispiel 1:

Durch Umsetzung von 87,9 g Glycerin, wasserfrei Ph.Eur, mit 12,0 g des Emulgators Polyoxy-ethylen-sorbitan-mono-oleat (E 433) und 0,1 g Sporopollenin (Kat. Nr. 16867 bei der Polysciences Europe GmbH, Eppelheim oder zu beziehen bei der RTC GmbH, Nordsehl) wurde unter gutem Rühren und Erwärmen auf eine Temperatur zwischen 40 °C - 50 °C für einen Zeitraum von etwa 30 Minuten eine Sporopolleninlösung hergestellt, welche nach dem Abkühlen auf Raumtemperatur in Lösung blieb. Diese Mischung zeigte auch innerhalb von 6 Wochen keine Entmischung der Komponenten.

### Herstellungsbeispiel 2:

Beispiel 1 wurde wiederholt, allerdings als Lösemittel 91,6 g Propylenglykol, Ph.Eur und als Emulgator 7,9 g Polyoxy-ethylen-sorbitan-mono-oleat (E 433) und 0,5 g Sporopollenin eingesetzt. Nach der Abkühlung erhielt man eine entsprechende leicht gelb gefärbte Lösung, die sich nach 2 Wochen entmischt.

### Herstellbeispiel 3 (Referenz beispiece):

Beispiel 1 wurde wiederholt, allerdings als Lösemittel 91,9 g Wasser eingesetzt und als Emulgator 8,0 g Brij 35 P Polyoxyethylenlaurylether (Laureth-23) zugesetzt sowie 0,1 g Sporopollenin. Nach derAbkühlung erhielt man eine entsprechende leicht gelb gefärbte Lösung, die auch nach 6 Wochen keine Entmischung zeigte.

### Herstellbeispiel 4:

Durch Rühren, Erwärmen auf etwa 40°C und anschließendes Abkühlen wurde eine Mischung aus 0,1 g-Sporopollenin mit einer Mischung von 88,0 g Ethanol 90%ig und 11,9 g des Emulgators Myrj 45 (Polyoxyethylen-8-Stearat) erhalten. Die Lösung zeigte auch nach 6 Wochen keine Entmischung.

### Herstellbeispiel 5:

Aus 5 mg Sporopollenin entsprechend 0,12 Gew.-%, 483 mg des Emulgators Polyoxy-ethylen-sorbitan-mono-oleat. (E 433) = 11,77 Gew.-% sowie 3617 mg Miglyol 812 Neutralöl (gemischsäuriges Triglycerid der fraktionierten Cocosfettsäuren C₈-C₁₀; erhältlich bei der Degussa AG, Werk Troisdorf) = 88,11 Gew.-% wurde durch Rühren, Erwärmen eine Lösung erstellt, die wenigstens 2 Tage unentmischt blieb.

### Herstellbeispiel 6:

Aus 4 mg Sporopollenin entsprechend 0,48 Gew.%, 146 mg des Emulgators Polyoxy-ethylen-sorbitan-mono-oleat (E 433) = 17,40 Gew.-% sowie 689 mg destilliertes Wasser = 82, 12 Gew.-% wurde durch Rühren, Erwärmen eine Lösung erstellt, die nach etwa 2 Tagen eine Teil-Entmischung zeigte in Form von Sporopolleninresten am Boden, die durch erneutes Erwärmen und Rühren aber wieder gelöst wurde.

### Herstellungsbeispiel 7:

Aus 4 mg Sporopollenin entsprechend 1,29 Gew.-%, 43 mg des Emulgators Polyoxy-ethylen-sorbitan-mono-oleat (E 433) = 13,87 Gew.-% sowie 263 mg *iso*-Propanol 70%ig = 84,84 Gew.-% wurde durch Rühren, Erwärmen eine Lösung erstellt, die nach etwa 2 Tagen eine Teil-Entmischung zeigte, die aber durch erneutes Erwärmen und Rühren wieder gelöst werden konnte

### Herstellbeispiel 8:

Aus 8 mg Sporopollenin entsprechend 0,62 Gew.%, 137 mg des Emulgators Polyoxy-ethylen-sorbitan-mono-oleat (E 433) = 10,54 Gew.-% sowie 1155 mg Glycerin DAB = 88,84 Gew.% wurde durch Rühren, Erwärmen eine Lösung erstellt, die nach 3 Tagen eine Teil-Entmischung zeigte in der Form, dass das gesamte Sporopollenin in dem Emulgator dispergiert war.

### Herstellbeispiel 9:

Aus 6 mg Sporopollenin entsprechend 0,62 Gew.-%, 66 mg des Emulgators Polyoxy-ethylen-sorbitan-mono-oleat (E 433) = 10,54 Gew.% sowie 394 mg Propylenglycol DAB = 88,64 Gew.-% wurde durch Rühren, Erwärmen eine Lösung erstellt, die nach ca. 3 Tagen eine Teil-Entmischung insofern zeigte, dass das Sporopollenin zum Teil im Emulgator gelöst ist, sich jedoch ein Bodensatz an nicht gelöstem Sporopollenin gebildet hat.

### Herstellbeispiel 10 (Referenz beispiel):

Aus 3 mg Sporopollenin entsprechend 0,74 Gew.-%, 46 mg des Emulgators Brij 35 P (Laureth-23) = 11,30 Gew.% und 358 mg Ethanol 90%ig wurde durch Rühren, Erwärmen eine Lösung erstellt, die mach ca. 2 Tagen eine Teil-Entmischung zeigte, die Sporopolleninreste am Boden aber durch erneutes Erwärmen und Rühren wieder gelöst werden konnten.

### Herstellbeispiel 11 (Referenz beispiel):

Aus 7 mg Sporopollenin entsprechend 1,08 Gew.-%, 46 mg des Emulgators Brij 35 P (Laureth-23) =7,11 Gew.-% sowie 594 mg *iso*-Propanol 70%ig = 91,81 Gew.% wurde durch Rühren, Erwärmen eine Lösung erstellt, die nach ca. 2 Tagen eine Teil-Entmischung zeigte, die Sporopolleninreste am Boden aber durch erneutes Erwärmen und Rühren wieder gelöst werden konnten.

### Herstellbeispiel 12 (Referenz beispiel):

Aus 3 mg Sporopollenin entsprechend 0,34 Gew.%, 78 mg des Emulgators Brij 35 P (Laureth-2,3) = 8,99 Gew.-% sowie 787 mg destillierten Wassers = 91,81 Gew.-% wurde durch Rühren, Erwärmen eine Lösung erstellt, die nach ca. 2 Tagen eine Teil-Entmischung zeigte, die Sporopolleninreste am Boden aber durch erneutes Erwärmen und Rühren wieder gelöst werden konnten.

### Herstellbeispiel 13 (Referenz beispiel):

Aus 3 mg Sporopollenin entsprechend 0,63 Gew.-%, 77 mg des Emulgators Brij 35 P (Laureth-23) = 16,04 Gew.% sowie 400 mg Propylenglycol = 83,33 Gew.-% wurde durch Rühren, Erwärmen eine zähe, viskose Lösung erstellt.

### Herstellbeispiel 14 (Referenz beispiel):

Aus 3 mg Sporopollenin entsprechend. 0,61 Gew.%, 68 mg des Emulgators Myrj 45 (Polyoxyethylen-8-Stearat) = 13,85 Gew.-% sowie 420 mg Ethanol 90%ig = 85,54 Gew.% wurde durch Rühren und Erwärmen eine Lösung erstellt.

### Herstellbeispiel 15:

Aus 5 mg Sporopollenin entsprechend 0,38 Gew.%, 180 mg des Emulgators Myrj 45 (Polyoxyethylen-8-Stearat) = 13,49 Gew.-% sowie 1149 mg destill. Wasser = 86,13 Gew.% wurde durch Rühren und Erwärmen eine Lösung erstellt.

### Herstellbeispiel 16:

Aus 9 mg Sporopollenin entsprechend 0,65 Gew.%, 65 mg des Emulgators Myrj 45 (Polyoxyethylen-8-Stearat) = 4,67 Gew.% sowie. 1318 mg destill. Wasser = 94,68 Gew.% wurde durch Rühren, Erwärmen eine gelbliche, viskose Lösung erzeugt.

### Herstellbeispiel 17:

Aus 2 mg Sporopollenin entsprechend 0,58 Gew.-%, 20 mg des Emulgators Myrj 45 (Polyoxyethylen-8-Stearat) = 5,80 Gew.% sowie 323 mg Propylenglycol = 93,62 Gew.-% wurde durch Rühren, Erwärmen eine gelbliche, viskose Lösung erzeugt.

### Galenikbeispiel 1 (Öl):

Durch Vermischen von 5g der erfindungsgemäßen Sporopolleninlösung gemäß Herstellungsbeispiel 1 und 75 g Miglyol 812-Neutralöl und 20 g Paraffinöl wurde ein Öl hergestellt, welches z.B. topisch sowohl kosmetisch als auch pharmazeutisch verabreicht werden konnte. Falls gewünscht, können Duftstoff oder Parfüms zugesetzt werden, wobei der Anteil an Paraffinöl entsprechend reduziert wird

### Galenikbeispiel 2 (Creme mit Sporopolleninlösung): .

6 g Sporopolleninlösung (Zusammensetzung 92,25 % Glycerin, wasserfrei, Ph.Eur., 7,60 % Polyoxy-ethylen-sorbitan-mono-oleat, 0,15 % Sporopollenin) und 493 g Basiscreme DAC (Zusammensetzung s. Schöffling a.a.O., S. 321). Die ca. 50 °C warme Lösung wird unter Rühren zur Basiscreme DAB gegeben. Nach Abkühlung auf Raumtemperatur unter Rühren wird die Lösung soweit evaporiert, bis die gewünschte Konsistenz erreicht ist und danach erneut homogenisiert. Man erhält eine blass-gelblich Creme.

### Galenikbeispiel 3 (Creme mit festem Sporopollenin):

### Sporopollenin und Lipoid SLM 2005 Basis

2 mg Sporopollenin entsprechend 0,56 Gew.-%, 211 mg Ethanol 90%ig = 58,61 Gew.-% sowie 147 mg Lipoid SLM 2005 (Hautpflegecreme auf Lecithin-/Glycerinbasis, Produkt der Lipoid GmbH, Ludwigshafen) = 40,83 Gew.%.

Sporopollenin wird bei 50 °C in Ethanol suspendiert, diese Suspension wird unter Rühren zu Lipoid SLM 2005 gegeben. Nach Abkühlung auf Raumtemperatur wird die Lösung soweit evaporiert, bis die gewünschte Konsistenz erreicht ist. Man erhält eine blass-gelbliche Creme (170 mg, Konzentration an Sporopollenin 1,18 %), welche ethanolisch riecht. Wahlweise lassen sich auch noch Verdickungsmittel (z. B. Xanthan) mit einarbeiten, um die Viskosität zu beeinflussen.

### Galenikbeispiel 4 (Creme)

### Sporopolleninlösung und Basiscreme DAC

5 mg Sporopollenin entsprechend 0,61 Gew.%, 417 mg Ethanol 90%ig = 50,73 Gew.-% und 400 mg Basiscreme DAC = 48,66 Gew.-%.

Sporopollenin wird bei 50 °C in Ethanol suspendiert, diese Suspension wird unter Rühren zur Basiscreme DAC gegeben. Nach Abkühlung auf Raumtemperatur unter Rühren wird die Lösung soweit evaporiert, bis die gewünschte Konsistenz erreicht ist und erneut homogenisiert. Man erhält eine blass-gelbliche Creme, welche noch leicht ethanolisch riecht.

### Anwendungsbeispiel 1:

Eine gemäß Herstellungsbeispiel 1 hergestellte Sporopolleninlösung wurde auf ihre Hautverträglichkeit hin überprüft. Hierbei wurde nach den GLP Richtlinien und den Empfehlungen der COLIPA Arbeitsgruppe (Walker A.P. et al: Test Guidelines for Assessment of Skin Compatibility of Cosmetic Finished Products in Man, Food and Chemical Toxicology 34, 1996, 651-660) vorgegangen.

Die Untersuchung erfolgte an 50 Testpersonen, d.h. 21 Hautgesunden, 11 Atopikern, 2 Allergikern und 16 Personen mit empfindlicher Haut im Alter von 18 - 65 Jahren.

Das Produkt gemäß Herstellungsbeispiel 1 wurde unverdünnt auf den Rücken des Probanden mit Hilfe von quadratischen Kunststoffkammern für 48 Stunden unter Okklusion appliziert. Als Positivkontrolle diente der Modellschadstoff Natriumlaurylsulfat in einer Konzentration von 1 % in Wasser. Als negative Kontrolle diente Wasser selbst.

Die Bewertung der Testreaktion erfolgte nach 48 und 72 Stunden nach dem modifizierten Draize-Test. Die gefundenen Testergebnisse zeigten, dass unter den Testbedingungen die 1 %-ige SDS-Lösung bei 14 Testpersonen zu einer positiven Reaktion führte. Die negative Kontrolle zeigte wie erwartet bei keiner der Personen eine Reaktion. Die erfindungsgemäße Lösung zeigte aufgrund der Testergebnisse und der gewählten Testbedingungen hinsichtlich einer eventuell hautreizenden Wirkung eine unbedenkliche Einstufung.

### Anwendungsbeispiel 2:

Bestimmung der Faltentiefe im Gesicht mittels der optischen 3D-in-vivo-Hautmeßsystemmethode "PRIMOS".

### Messung der Faltentiefe

Die Bestimmung erfolgt mit dem optischen 3D-in-vivo-Hautmeßsystem "PRIMOS" (Phaseshift-Rapid In-vivo- Measurement Of Skin - Hersteller: GFMeßtechnik GmbH, 14513 Teltow/Berlin). Das optische 3D-vivo-Hautmeßsystem basiert auf der sogenannen Streifenprojektionstechnik. Bei der Streifenprojektionstechnik wird über eine Projektionsoptik ein paralleles Streifenmuster auf die Hautoberfläche projiziert und über eine Abbildungsoptik auf dem Chip einer CCD-Matrixkamera abgebildet. Durch feinste Höhenunterschiede auf der Haut werden die parallelen Streifen ausgelenkt. Die Größe der Auslenkung stellt sowohl ein qualitatives als auch ein quantitatives Maß für das jeweilige Höhenprofil dar. Durch den festen Bezugswinkel zwischen Projektionsoptik und Kamerasystem lassen sich Profilhöhen durch einfache Triangulationsgleichungen bestimmen. Die mögliche Profilhöhenauflösung wird über die verwendete effektive Wellenlänge λ_{eff} durch die zu messende Höhendifferenz bestimmt.

Zur Beschreibung der Faltentiefe wird der Parameter Rmax (Din 4768/1) gewählt. Rmax ist die maximale Rauhtiefe, d.h. der Abstand zwischen der Linie der Erhebung und der Linie der Vertiefung innerhalb der Messstrecke.

### Versuchsdurchführung

Die Probandinrien wurden über Tragweite und Bedeutung der Studie aufgeklärt. Anschließend unterzeichneten sie eine Einverständniserklärung. Ihre Auswahl erfolgte nach folgenden Kriterien:
Einschlusskriterien:
   - Frauen älter als 18 Jahre
   - klinisch gesund
   - deutlich sichtbare Falten im Augenbereich
Ausschlusskriterien:
   - Hauterkrankungen
   - Schwangerschaft

Die Probandinnen konnten jederzeit ohne Angabe von Gründen aus der Studie ausscheiden. Für die Anwendung erhielten alle Testteilnehmer eine detaillierte Anwendungsanleitung.
Die Testpersonen wurden angewiesen, 3 Tage vor Beginn der Prüfung und während der gesamten Testzeit keine anderen Extema im Gesicht aufzutragen.
Während der Produktanwendung durfte zur Reinigung in den Testfeldern nur Wasser oder ein mildes Syndet (Eubos ^{®} flüssig - blau; Hersteller: Dr. Hobein, D-53340 Meckenheim-Merl, Germany) angewendet werden.

Die Messungen erfolgten vor der ersten Applikation des Produktes an genau definierten Stellen im Gesicht (Augenbereich: Krähenfüße). Weitere Messungen erfolgten nach ein-und zweiwöchiger Anwendung des Produktes 8 bis 12 Stunden nach der täglichen Applikation (Einklimatisierungszeit: 30 min, Raumtemperatur: 20 ± 1°C, relative Luftfeuchtigkeit: 50 ± 10 %). Das Produkt wurde von den Probandinnen einmal täglich (abends im Gesicht) in einer Weise angewendet, die möglichst weitgehend der Anwendung der zu prüfenden Produkte durch den Verbraucher entspricht. Die linke Gesichtshälfte wurde mit dem Testprodukt behandelt, die rechte blieb unbehandelt und diente als Kontrolle.

### Biometrie

Die Messdaten werden zentral nach eingehender Plausibilitätsprüfung und Qualitätssicherung in den Rechner übernommen. Die Auswertung wird mit Hilfe der Software WinSTAT^{®} Add-in for Excel - R.K. Fitch, Germany durchgeführt. Für die statistische Analyse wurde der Wilcoxon matched pairs signed rank test gewählt. p<0,05 gilt als Hinweis für einen statistischen Unterschied.

### Ergebnisse

### Reduktion der Faltentiefe

Die regelmäßige Anwendung des Testproduktes führte nach 14 Tagen im Gesicht (Augenfalten) zu einer statistisch signifikanten Verminderung (p<0,05) der Faltentiefe im Vergleich zur Kontrolle. Die Verringerung der Faltentiefe betrug im Mittel (Angaben in % bezogen auf den Ausgangswert):

| | | |
|---|---|---|
| | **Produkt** | **unbehandelt** |
| Tag 7 | 4,7 | -1,3 |
| Tag 14 | 8,9* | -1,5 |

| | | |
|---|---|---|
| p<0,05 versus unbehandelt | | |

### Toxizität:

Die orale Toxizität der erfindungsgemäßen Sporopolleninenthaltenen Zusammensetzungen, am Beispiel des sporopolleninenthalienen Algenextrakts Caelico®, (Vertrieb durch RTC GmbH, Nordsehl), suspendiert in 0,8% wässrigen Hydroxypropylmethylcellulosege wurde an CD® Ratten nach der OECD Richtlinie 423 und der EC Richtlinie L 248: B1 tris (ATC-verfahren) durchgeführt ( entspre-chend den US Food and Drug Administration Good Laboratory Practice Regulations 21 Code of federal Regulations, Part 58, entsprechned den Japanese guidelines for Non-clinical studies of drugs, Manual 1995, Guidelines for Tocicity studies of drugs, Japanese Ministry of Health and Welfare wo bei oraler Einnahme sowohl der Placebolevel als auch der LD50 level bei mehr als 2 g pro Kg Körpergewicht lag..

### Galenikbeispiel 5 (Futtermittel mit festem Sporopollenin)

0,1 g Sporopollenin gemäß Herstellungsbeispiel 1 und 9,9 g (bzw. 4,95 g) an geeigneter Trägerstoff (Lactose oder Maisstärke) werden im Mörser vermischt und solange verreiben, bis eine homogene Mischung erreicht ist. Der Gehalt an Sporopollenin beträgt 1 % (bzw. 2 %).

### Beispiel 6 (Getränk mit gelöstem Sporopollenin)

| Zutaten | Menge in g |
|---|---|
| Wasser | 920 |
| Glucose Sirup DE 70 | 90 |
| Orangensaftkonzentrat 65 °Bx | 4,15 |
| Zitronensäure (wasserfrei) | 3,5 |
| | |
| Sporopollenin gemäß Herstellungsbeispiel 1 | 2 |
| Modifizierte Stärke | 1,37 |
| Grapefruitsaftkonzentrat 60 °Bx | 1,25 |
| | |
| Sonnenblumenöl | 1,1 |
| Zitronensaftkonzentrat 65 °Bx | 0,55 |
| Carboxymethylzellulose | 0,74 |
| Beta Carotin | 0,5 |
| | |
| Natriumbenzoat 20%H₂O | 0,5 |
| Vitamin C | 0,5 |
| Xanthan | 0,46 |
| Kaliumsorbat | 0,1 |
| Acesulfam K | 0,1 |
| Aspartam | 0,1 |
| | |
| Summe | 1 Liter |

Die Herstellung des Getränks erfolgt in der Weise, daß Wasser, Glucose Syrup, Natriumbenzoat, dann die Saftkonzentrate und das Öl eingewogen werden, dann die Einwaage und Mischen der pulverförmigen Komponenten mit Ausnahme des beta Carotin erfolgt. Diese Pulvermischung wird zur flüssigen Phase unter Rühren bei ca. 5000 U/min zugefügt, 5 mal bei 220 bar homogneisiert, das Beta-Carotin zum Getränk hinzugefügt, bei bei 90°C für 10 min pasteurisiert und schließlich bei +4 °C gelagert.

Die Gehalte an Fruchtsaftkonzentraten, Zitronensäure und beta-Carotin können je nach Geschmacksrichtung variiert werden. Auch der Gehalt an Sporopollenin kann variiert werden. Die Süßstoffe und beta-Carotin sind nicht zwingend erforderlich, dann sollte entsprechend mehr Glucose Sirup verwendet werden.

### Schwermetallbindung bzw. Ausleiftung (Referenz beispiel):

Die nachstehenden AAS Messungen wurden an einem SPECTROFLAME - ICP P Spektrometer des Typs FLAMEFV04-38/043, S/N: 0213/90, Spannung:220 V/50 Hz. Leistung:5500 VA der SPECTRO ANALYTICAL INSTRUMENTS GmbH, Kleve durchgeführt.

### Anwendungsbeispiel 3 (Referenz beispiel):

Zu einer Lösung aus 11 mg Bleiacetat-Trihydrat (Merck, Nr. 107372), 11 mg Cadmiumnitrat-4 Hydrat (Riedel de Haen, Nr. 11714), 11 mg Arsen(V) oxid-Hydrat (Fluka, 11304) und 30 ml Wasser dest. werden 15 mg Sporopollenin gemäß Herstellbeispiel 1 (Versuch, 1) gegeben. Die Lösung wird für 2 Stunden gerührt und das Sporopollenin abfiltriert. Von dieser gefilterten Lösung wird 1 ml im Atom Absoptions Spektrometermessung verwendet. Vor der Messung wird die Lösung 1: 20 mit Wasser, dest. verdünnt.

### Anwendungsbeispiel 4 (Referenz beispiel):

Zu einer Lösung aus 11 mg Bleiacetat -Trihydrat, 11 mg Cadmiumnitrat-4 Hydrat, 11 mg Arsen(V)oxid-Hydrat und 30 ml Wasser dest. werden 15 mg Sporopollenin gemäß Herstellbeispiel 1 (Versuch 2) gegeben. Die Lösung wird für 2 Stunden gerührt und das Sporopollenin abfiltriert. Von dieser gefilterten Lösung wird 1 ml für die Atom Absoptions Spektrometermessung verwendet. Vor der Messung wird die Lösung 1 : 20 mit Wasser, dest. verdünnt.

### Messmethode:

Die Proben wurden verdünnt siehe Versuchsbeschreibung und die Messung am ICP-OES simultan durchgeführt ICP - OES (Inductively Coupled Plasma- Optical Emission Spectrometry) Für die Analyse von Spurenelementen in Lösungen in den Konzentrationsbereichen mg/l bis µg/l wird die Optische Emissionsspektrometrie mit dem induktiv gekoppeltem Plasma (ICP-OES) eingesetzt. Dieses Verfahren ermöglicht prinzipiell die gleichzeitige Bestimmung aller Metalle und einiger Nichtmetalle (bis zu 60 Elemente) aus angesäuerten, wässerigen Lösungen bis zu einem Gesamtgehalt an gelöster Substanz von ca. 10 g/l. Beim ICP-OES wird eine flüssige Probe zerstäubt und die Atome mittels eines induktiv gekoppelten Plasmas (Temperatur bis zu 10000 K) zur Aussendung von Licht angeregt. Das emittierte Licht wird mit einem Gitter zerlegt. Ein Elektronenvervielfacher als Detektor wandelt Licht in elektrische Signale um. Simultan messende Spektrometer sind in der Lage sehr viele Emissionslinien zum gleichen Zeitpunkt zu erfassen.

### Messergebnis:

| | Gehalt: 97,00% | | Gehalt 99,00% | | Gehalt: 99,50% | |
|---|---|---|---|---|---|---|
| | As (193,696 nm) | | Cd (226,502 nm) | | Pb (220,351 nm) | |
| | Ausgangs | | Ausgangs | | Ausgangs | |
| | konzentration | Gemessen | konzentration | Gemessen | konzentration | Gemessen |
| | (mg / ml) | (mg / ml) | (mg / ml) | (mg / ml) | (mg / ml) | (mg / ml) |
| Versuch 1 | 0,696 | 0,116 | 0,397 | 0,138 | 0,597 | 0,004 |
| Versuch 2 | 0,696 | 0,117 | 0,397 | 0,148 | 0,597 | 0,003 |

Die gefundenen Messwerte wurden schon entsprechend der Verdünnung umgerechnet.

Es wurde gefunden, dass As zu 83 % von Sporopollenin entfernt / abgebaut wird, Cd wird zwischen 62 % und 65 % von Sporopollenin entfernt / abgebaut, Pb wird zu 99 % von Sporopollenin entfernt / abgebaut.

## Patentansprüche

1. Sporopollenin enthaltende Zusammensetzung, **dadurch gekennzeichnet, dass** sie als lösung vorliegt und neben
- 0,01 bis 12 Gew.-%, vorzugsweise 0,1 bis 5 Gew.-%. Sporopollenin
- 0,01 bis 17 Gel.-%, vorzugsweise 0,1 bis 10 Gel.-% wenigstens eines Emulgatore, wobei der Emulgator eine hydrolisierbare Esterverbindung ist und
- 71,00 bis 99,98 Gew.-%, vorzugsweise 85 bis 99,80 Gel.-% wenigstens eines hydrophilen Lösemittels aufweist, wobei das hydrophile Lösemittel Waaser und / oder ein ggf. Wasser enthaltender Alkohol mit bis zu 22 Kohlenstoffatomen und / oder ein Ester mit 2 bis 33 Kohlenstoffatomen ist.

2. Zusammensetzung nach Anspruch 1, **dadurch gekennzeichnet, dass** der Emulgator eine hydrolisierbare Esterverbindung eines mehrwertigen Alkohols, insbesondere ein Polyoxyethylen-sorbitanester von Fettsäuren ist.

3. Sporopollenin enthaltende Zusammensetzung, **dadurch gekennzeichnet, dass** sie als Lösung vorliegt und neben
- 0,01 bis 12 Gew.-%, vorzugsweise 0,1 bis 5 Gew.-%,Sporopollenin
- 0,01 bis 17 Gew.-%, vorzugsweise 0,1 bis 10 Gew.-% wenigstens eines Emulgators, wobei der Emulgator ein hydrophiler nichtionischer Emulgator, insbesondere mit einem HLB-Wert von 10 -17 ist.
und
- 71,00 bis 99,98 Gew.-%, vorzugsweise 85 bis 99,80 Gew.-% wenigstens eines hydrophilen Lösemittels aufweist, wobei das hydrophile Lösemittel Wasser und / oder ein ggf. Wasser enthaltender Alkohol mit bis zu 22 Kohlenstoffatomen und 1 oder ein Ester mit 2 bis 33 Kohlenstoffatomen ist.

4. Biologische Zusammensetzungen, enthaltend zeine Sporopolleninlösung nach Ansprüchen 1 bis 3 zusammen mit üblichen Hilfs- und Zusatzstoffen.

5. Kosmetische Zusammensetzungen, enthaltend eine Sporopolleninlösung nach Ansprüchen 1 bis 3 in Form eines Öls, Salbe, Creme, Gel, eingebettet in Liposomen. Milch oder als fester Stift.

6. Zusammensetzung nach Anspruch 5, **dadurch gekennzeichnet, dass** Sporopollenin als Feststoff eingesetzt wird.

7. Pharmazeutische Zusammensetzungen, enthaltend eine Spotopolleninlösung nach Ansprüchen 1 bis 3, in Form eines Öls, einer Creme, oder verkapselt in Weich- oder Hartgelabnekapseln.

8. Zusammensetzung nach Anspruch 7, **dadurch gekennzeichnet, dass** Sporopollenin als Feststoff eingesetzt wird.

9. Zusammensetzungen nach Anspruch 8, **dadurch gekennzeichnet, dass** die Gelatinekapsein Sporopollenin als Feststoff enthält.

10. Verwendung der Sporopollenin enthaltenden Lösungen nach Anspruch 1 bis 3 zur Herateltung eines Mittels zur kosmetischen Behandlung der Haut, insbesondere der Gesichtshaut, gegen attersbedingte und / oder (sonnen)lichtbedingte Heutfaltenbildung.

11. Verwendung der Sporopollenin enthaltenden Lösung nach Anspruch 1 bis 3 zur Herstellung eines Nahrungs(ergänzungs)mittel für Wirbeltiere.

12. Verwendung nach Anspruch 11, **dadurch gekennzeichnet, dass** Sporopollenin als Feststoff eingesetzt wird.

## Claims

1. A composition containing sporopollenin, **characterized in that** it is present as a solution and that, aside from
• 0.01 % to 12% by weight, preferably 0.1 % to 5% by weight, of sporopollenin,
• it also contains 0.01 % to 17% by weight, preferably 0.1 % to 10% by weight, of at least one emulsifier and
• 71.00% to 99.98% by weight, preferably 85% to 99.80% by weight, of at least one hydrophilic solvent, wherein the hydrophilic solvent is water and/or an alcohol, optionally containing water, having up to 22 carbon atoms and / or an ester having 2 to 33 carbon atoms.

2. The composition according to Claim 1, **characterized in that** the emulsifier is a hydrolyzable ester compound, especially a hydrolyzable ester compound of a polyvalent alcohol, particularly a polyoxyethylene sorbitan ester of fatty acids.

3. A composition containing sporopollenin, **characterized in that** it is present as a solution and that, aside from
• 0.01 % to 12% by weight, preferably 0.1 % to 5% by weight, of sporopollenin,
• it also contains 0.01 % to 17% by weight, preferably 0.1% to 10% by weight, of at least one emulsifier, wherein the emulsifier is a hydrophilic non-ionic emulsifier, especially having an HLB value of 10 to 17 and
• 71.00% to 99.98% by weight, preferably 85% to 99.80% by weight, of at least one hydrophilic solvent, wherein the hydrophilic solvent is water and/or an alcohol, optionally containing water, having up to 22 carbon atoms and / or an ester having 2 to 33 carbon atoms..

4. Biological compositions containing a sporopollenin solution according to Claims 1 to 3, together with commonly employed auxiliaries and additives.

5. Cosmetic components, containing a sporopollenin solution according to Claims 1 to 3, in the form of an oil, an ointment, a cream, a gel embedded in liposomes, a milk or else as a solid stick.

6. The composition according to Claim 5, **characterized in that** sporopollenin is used as a solid.

7. Pharmaceutical compositions containing a sporopollenin solution according to Claims 1 to 3, in the form of an oil, a cream or encapsulated in soft or hard gelatin capsules.

8. The composition according to Claim 7, **characterized in that** sporopollenin is used as a solid.

9. Compositions according to Claim 8, **characterized in that** the gelatin capsule contains sporopollenin as a solid.

10. The use of solutions containing sporopollenin according to Claims 1 to 3 for the production of an agent for the cosmetic treatment of the skin, especially facial skin, against age-related and/or (sun)light-related wrinkle formation.

11. The use of solutions containing sporopollenin according to Claims 1 to 3 as nutritional supplements for vertebrates.

12. The use according to Claim 11, **characterized in that** sporopollenin is used as a solid.

## Revendications

1. Composition contenant de la sporopollénine, **caractérisée en ce qu'**elle se présente sous forme de solution et contienne
- de 0,01 à 12 % du poids, de préférence de 0,1 à 5 % du poids, de sporopollénine et
- de 0,01 à 17 % du poids, de préférence de 0,1 à 10 % du poids, d'au moins un émulsifiant, cependant que l'émulsifiant est un composé hydrolysable à base d'ester, ainsi que
- de 71,00 à 99,98 % du poids, de préférence de 85 à 99,80 % du poids, d'au moins un solvant hydrophile, cependant que le solvant hydrophile est de l'eau et /ou un alcool pouvant contenir de l'eau et doté de jusqu'à 22 atomes de carbone et / ou un ester doté de 2 à 33 atomes de carbone.

2. Composition selon la revendication n° 1, **caractérisée en ce que** l'émulsifiant est un composé hydrolysable à base d'ester et de polyalcool, en particulier d'un ester d'acides gras de polyoxyéthylène sorbitol.

3. Composition contenant de la sporopollénine, **caractérisée en ce qu'**elle se présente sous forme de solution et contienne
- de 0,01 à 12 % du poids, de préférence de 0,1 à 5 % du poids, de sporopollénine et
- de 0,01 à 17 % du poids, de préférence de 0,1 à 10 % du poids, d'au moins un émulsifiant, cependant que l'émulsifiant est un émulsifiant hydrophile non ionique, en particulier présentant une valeur HLB entre 10 et 17, ainsi que
- de 71,00 à 99,98 % du poids, de préférence de 85 à 99,80 % du poids, d'au moins un solvant hydrophile, cependant que le solvant hydrophile est de l'eau et /ou un alcool pouvant contenir de l'eau et doté de jusqu'à 22 atomes de carbone et / ou un ester doté de 2 à 33 atomes de carbone.

4. Compositions biologiques, qui contiennent une solution de sporopollénine selon les revendications n° 1 à n° 3, ainsi que des consommables et additifs usuels.

5. Compositions cosmétiques, qui contiennent une solution de sporopollénine selon les revendications n° 1 à n° 3 sous forme d'huile, de pommade, de crème, de gel, incorporée dans des liposomes, sous forme de lait ou de stick solide.

6. Composition selon la revendication n° 5, **caractérisée en ce que** de la sporopollénine est utilisée sous forme solide.

7. Compositions pharmaceutiques, qui contiennent une solution de sporopollénine selon les revendications n° 1 à n° 3 sous forme d'huile, de crème ou encapsulée dans des capsules de gélatine dure ou molle.

8. Composition selon la revendication n° 7, **caractérisée en ce que** de la sporopollénine est utilisée sous forme solide.

9. Composition selon la revendication n° 8, **caractérisée en ce que** les capsules de gélatine contiennent de la sporopollénine sous forme solide.

10. Utilisation des solutions contenant de la sporopollénine selon les revendications n° 1 à n° 3 pour la préparation d'un produit de traitement cosmétique de la peau, en particulier de la peau du visage, pour prévenir la formation de rides de la peau qui soient conditionnées par l'âge et / ou la lumière (du soleil).

11. Utilisation de la solution contenant de la sporopollénine selon les revendications n° 1 à n° 3 pour la préparation d'un produit (de complément) alimentaire pour des vertébrés.

12. Utilisation selon la revendication n° 11, **caractérisée en ce que** la sporopollénine est utilisée sous forme solide.
